# EUROPÄISCHE PATENTSCHRIFT

(11) **EP 1 593 399 B1**
(45) Veröffentlichungstag und Bekanntmachung des Hinweises auf die Patenterteilung: **25.08.2010**
(21) Anmeldenummer: 05009917.5
(22) Anmeldetag: 06.05.2005
(51) Int. Cl.: A61L 15/32, A61L 15/20, A61L 15/46

(54) **Bioresorbierbare Wundauflage auf Kollagen-Basis**
Bioresorbable wound dressing based on collagen
Pansement pour plaie biorésorbable à base de collagène

(30) Priorität: 07.05.2004 DE 102004022645
(43) Veröffentlichungstag der Anmeldung: 09.11.2005
(73) Patentinhaber: Resorba Wundversorgung GmbH & Co. KG, 90475 Nürnberg (DE)
(72) Erfinder: Hiltner, Claus M., 90408 Nürnberg (DE); Sorg, Karl-Heinz, Dr., 93333 Neustadt (DE); Alblas, Ton Pieter, 45549 Sprockhövel (DE)
(74) Vertreter: Tomerius, Isabel

(56) Entgegenhaltungen:
- EP-A- 0 314 109
- WO-A-00/33829
- WO-A-03/004013
- WO-A-2006/111347
- US-A1- 2003 187 036

## Beschreibung

Die Erfindung betrifft eine bioresorbierbare Wundauflage auf der Basis von Kollagen. Kollagen ist als Gerüsteiweiß des Bindegewebes Bestandteil des Körpers von Säugetieren. Kollagen wird üblicherweise aus den Sehnen und/oder Häuten von Rindern, Schweinen oder Pferden gewonnen. Abhängig von seiner Herkunft und seiner Struktur wird Kollagen in verschiedene Typen eingeteilt. Allen Kollagentypen ist eine Tripelhelix-Struktur gemeinsam, die aus drei Polypeptidketten gebildet wird.

Als körpereigenes Material ist Kollagen ausgezeichnet körperverträglich und gut resorbierbar und in dieser Hinsicht körperfremden, synthetischen Materialien wie Cellulose deutlich überlegen. Entsprechend findet Kollagen seit längerem Anwendung als Wundauflage zur lokalen Blutstillung, als Material zur Auskleidung von Gewebs- und Knochendefekten, als Hautersatz bei Läsionen und als Hautabdeckung bei großflächigen Verbrennungen. Auch als resorbierbares Nahtmaterial wird Kollagen eingesetzt.

Neben seiner sehr guten Körperverträglichkeit und Resorbierbarkeit hat Kollagen gegenüber anderen Materialien zudem den Vorteil, dass es blutstillend wirkt. Ein Anwendungsgebiet des Kollagens liegt daher in der Versorgung von Wunden, insbesondere solchen Wunden, bei denen ein Teil der dermalen Strukturen oder auch der unter der Haut liegenden Strukturen in Mitleidenschaft gezogen wurde. Kollagen wird sowohl zur Behandlung akuter als auch chronischer Wunden eingesetzt. Als chronische Wunden werden üblicherweise solche bezeichnet, die selbst nach einer Periode von 4 bis 6 Wochen keine Tendenz zur Heilung zeigen. Beispiele chronischer Wunden sind
- Ulcus cruris (z. B. auf Grund venöser oder arterieller Erkrankungen)
- das diabetische Fußsyndrom
- Dekubitus, d. h. ein Bereich der Haut, in dem durch übermäßigen Druck eine Wunde entstanden ist, mit Nekrose und Ulceration, oft mit Beteiligung der dermalen und subdermalen Gewebe.

Es ist bekannt, dass Kollagen die Heilung akuter und chronischer Wunden fördern kann. In einem ersten Schritt kommt es dabei zur Thrombozytenaggregation an den Kollagenfibrillen. Der Blutfluss in der Wunde wird gestillt und die Wunde mit einem Koagel verschlossen. Außerdem wechselwirkt das Kollagen mit Strukturen der Gefäßwände und den Bindegewebsproteinen und aktiviert an der Wundheilung beteiligte Körperzellen, insbesondere die Fibroblasten. Auf diese Weise wird die Festigkeit des Koagels erhöht. Im letzten Stadium der Wundheilung wird das Kollagen durch eingewanderte Macrophagen und Kollagenase vom Körper resorbiert, die Wunde wird mit körpereigenem Material aufgefüllt, und die Wundoberfläche wird endgültig geschlossen und geglättet.

Die Wundheilung kann jedoch unter anderem durch Infektion der Wunde stark beeinträchtigt oder verhindert werden. Zur Verminderung von Infektionen sind daher Wundauflagen vorgeschlagen worden, die eine mikrobizide Substanz enthalten. In der US-A-6,468,521 beispielsweise wird eine Wundauflage beschrieben, deren hydrophiler polymerer Träger mit einem Silber-Amin-Komplex imprägniert ist. Die Silberverbindung besitzt bakteriostatische und fungistatische Eigenschaften. Als Trägermaterial ist unter anderem Kollagen genannt. Offenbar sind solche silberhaltigen Verbände jedoch problematisch in der Anwendung (vergleiche S. Coerper, G. Gottwald, S. Beckert und H. D. Becker, "Wundheilung und -behandlung 2004 - Der aktuelle Stand" in ZfW Nr. 1/04, Seiten 20 bis 23; Seite 21, rechte Spalte).

Während die gute Resorbierbarkeit von Kollagen im allgemeinen als Vorteil anzusehen ist, kann eine zu schnelle Resorption des Kollagens bei der Wundbehandlung auch Nachteile mit sich bringen. Die Schnelligkeit der Resorption von Kollagen im Körper hängt einerseits von dessen Struktur und insbesondere vom Vernetzungsgrad ab, wird aber andererseits auch durch den Applikationsort entscheidend beeinflusst. In stark durchblutetem Gewebe beispielsweise kann Kollagen innerhalb von 2 bis 3 Tagen vollständig aufgelöst sein, während unter anderen Bedingungen die Resorption 2 bis 6 Wochen in Anspruch nehmen kann. In der DE 19503336 C2 wird beispielsweise beschrieben, dass eine Arzneimittelzubereitung mit Depotwirkung, bei der das Arzneimittel auf einem mit Chlorhexidinhydrochlorid versetzen Kollagenträger verabreicht wird, im Körper bereits nach 30 Minuten vollständig zerfallen ist. Bei Wundauflagen ist es aber häufig wünschenswert, dass diese mindestens 72 Stunden auf der Wunde verbleiben. Zu häufige Wundverbandwechsel bergen nicht nur die Gefahr, dass die Wunde wieder aufgerissen wird, sondern erhöhen auch die Gefahr von Infektionen. Es besteht daher ein Bedarf an einer Wundauflage aus Kollagen, die auch in gut durchblutetem Gewebe über einen Zeitraum von mindestens 3 Tagen nicht vollständig resorbiert wird.

**Aufgabe** der Erfindung ist es entsprechend, eine resorbierbare Wundauflage auf der Basis von Kollagen zur Verfügung zu stellen, welche bei guter Körperverträglichkeit und guter Resorbierbarkeit über einen Zeitraum von einigen Tagen im Körper nicht vollständig aufgelöst wird und welche einen zuverlässigen Schutz vor Infektionen gewährleistet.

Die Lösung dieser Aufgabe gelingt mit der bioresorbierbaren Wundauflage gemäß Anspruch 1. Bevorzugte Ausführungsformen sind in den Unteransprüchen beschrieben.

Die Erfindung betrifft also eine bioresorbierbare Wundauflage auf der Basis von Kollagen, welche dadurch gekennzeichnet ist, dass sie einen Gehalt an einem linearen polymeren Biguanid und/oder einem wasserlöslichen Salz desselben aufweist. Als bevorzugtes Biguanid wird Polyhexamethylenbiguanid (PHMB) eingesetzt.

Lineare polymere Biguanide und insbesondere Polyhexamethylenbiguanid sind gut verträgliche Verbindungen, die für ihre gute mikrobizide Wirkung bekannt sind. Sie besitzen ein breites Wirkungsspektrum gegen eine Vielzahl von Bakterien, Fungi und sogar einige Viren. Die linearen polymeren Biguanide sind bereits in geringer Konzentration hoch wirksam und zudem über einen breiten pH-Bereich einsetzbar. Sie verlieren ihre mikrobizide Wirksamkeit auch bei hoher Eiweißbelastung nicht und können ohne Wirkungsverlust in Anwesenheit von Blut oder Eiweiß eingesetzt werden, was insbesondere bei der Wundbehandlung einen großen Vorteil darstellt. Zudem sind die linearen polymeren Biguanide in den üblicherweise eingesetzten Mengen praktisch nicht zytotoxisch. Dies ist für die Wundauflage sehr vorteilhaft, da so die Entstehung von Granulationsgewebe und damit letztlich die Epithelialisierung der Wunde nicht gestört wird.

Im Rahmen der Erfindung eingesetzt werden können alle linearen polymeren Biguanide, die eine keimtötende Wirkung besitzen. Bevorzugtes Biguanid ist jedoch Polyhexamethylenbiguanid (PHMB). Wie alle übrigen Biguanide kann es als solches oder in Form eines wasserlöslichen Salzes eingesetzt werden. Bevorzugt ist hier das Hydrochlorid, das beispielsweise in Form einer 20% wässrigen Lösung unter der Bezeichnung Vantocil® IB oder Cosmocil® CQ von der Firma Avecia, Manchester, GB, oder Frankfurt/Main, DE, erhältlich ist. Wenn im Folgenden allgemein von einem Biguanid oder PHMB die Rede ist, sollen gleichzeitig immer auch die Salze dieser Verbindungen mit angesprochen sein. Die Molekulargewichtsbereiche, in denen die Biguanide eingesetzt werden können, sind nicht besonders beschränkt. Vielmehr können alle diese Verbindungen mit den bislang üblichen Molekulargewichten verwendet werden. Im Falle von PHMB liegt das Molekulargewicht beispielsweise in einem Bereich von etwa 1500 bis 15000. Bevorzugt sind Molekulargewichte des PHMB unter 5000 und insbesondere unter 2900.

Die Einarbeitung des linearen polymeren Biguanids in das Kollagen kann auf verschiedene Weise geschehen. Sie kann während der Aufbereitung des Kollagens zu bestimmten Formen der bioresorbierbaren Wundauflage erfolgen oder im Anschluss daran. Das Biguanid kann als Feststoff in das Kollagen eingearbeitet werden oder in Form einer Lösung in einem geeigneten Lösemittel wie beispielsweise Wasser. Das Lösemittel kann anschließend aus der bioresorbierbaren Wundauflage abgedampft werden oder in diesem verbleiben, beispielsweise um eine feuchte Wundauflage zu ergeben.

Die linearen polymeren Biguanide oder deren Salze sind problemlos mit Kollagen kompatibel. Überraschend wurde zudem gefunden, dass der Zusatz des Biguanids die Resorption des Kollagens im Körper deutlich verlangsamen kann. Dies ist vermutlich darauf zurückzuführen, dass die bakterizide Wirkung des Biguanids die Ausbreitung von Mikroorganismen verhindert, welche das Enzym Kollagenase produzieren. Kollagenase bewirkt den Abbau des Kollagens, welches dadurch seine Konsistenz schnell verliert und geliert. Der Konsistenzverlust des Kollagens wiederum führt dazu, dass dieses seine Funktion als Wundauflage nicht mehr zufriedenstellend ausüben kann. Die Anwesenheit des linearen polymeren Biguanids mit seiner bakteriziden Wirkung in der erfindungsgemäßen resorbierbaren Wundauflage dagegen verlangsamt den Abbau des Kollagens, ohne dessen Resorption im Körper jedoch vollständig zu unterbinden. Der Strukturverlust des Materials tritt daher deutlich später ein. Selbst wenn die erfindungsgemäße bioresorbierbare Wundauflage in gut durchblutetem Gewebe verwendet wird, kann es dort ohne weiteres 72 Stunden oder länger verbleiben, ohne dass es während dieser Zeit vollständig resorbiert wird.

Die mikrobizide Wirkung, die der erfindungsgemäßen bioresorbierbaren Wundauflage durch den Gehalt an einem linearen polymeren Biguanid verliehen wird, wirkt sich zudem positiv auf das Gewebe aus, mit welchem die bioresorbierbare Wundauflage in Kontakt kommt. Einerseits führt die therapeutische Unterstützung zu einer deutlichen Beschleunigung der Heilung bei infizierten Wunden, anderseits ist die Wirkung prophylaktisch und verhindert die Infektion nicht infizierter Wunden beziehungsweise beugt bei kontaminierten und infizierten Wunden einer manifesten Infektion vor.

Die prophylaktische und/oder therapeutische Wirkung der erfindungsgemäßen bioresorbierbaren Wundauflage lässt sich noch dadurch erhöhen, dass sie einen Gehalt an wenigstens einem Tensid aufweist. Dieses Tensid führt zu einer Senkung der Oberflächenspannung im Gewebe, mit welchem die bioresorbierbare Wundauflage in Kontakt ist. Auf dem Gewebe gebildete Biofilme werden auf diese Weise aufgelöst. Außerdem wird bei gereinigtem Gewebe, auf welchem kein Biofilm vorhanden ist, verhindert, dass sich ein solcher Biofilm bildet. Biofilmen kommt im Falle der Wundversorgung eine besondere Bedeutung zu. Biofilme sind komplexe Strukturen, die entstehen, wenn Mikroorganismen sich auf einer Oberfläche festsetzen, wobei eine multizelluläre Struktur entsteht mit den genannten Zellen in einer von diesen Zellen produzierten extrazellulären biopolymeren Matrix. Diese Biofilme bilden sich gerne auf bestehenden Wundbelägen, welche sowohl Keimen aus der Hautflora als auch pathogenen Keimen gute Wachstumsbedingungen bieten und die Wundheilung erheblich verzögern. Auch die Ausbreitung von Wundinfektionen erfolgt üblicherweise über diese Wundbeläge, auf denen sich Mikroorganismen vermehren. Das Vorhandensein wenigstens eines Tensids in der erfindungsgemäßen bioresorbierbaren Wundauflage beeinträchtigt dagegen die Bildung und Ausbreitung von derartigen Biofilmen und Wundbelägen und verringert somit die Infektionsgefahr ganz erheblich.

Als Tensid in der erfindungsgemäßen bioresorbierbaren Wundauflage wird zweckmäßig ein nicht-ionisches Tensid, ein Amphotensid oder eine Kombination derselben eingesetzt. Derartige Tenside beeinträchtigen die antimikrobielle Wirkung des linearen polymeren Biguanids nicht, während bei anionischen Tensiden diese Gefahr besteht. Kombinationen von Biguaniden mit Tensiden sind im Stand der Technik grundsätzlich bekannt. Häufig wird beispielsweise PHMB in Kombination mit einem Polyethylenglycol eingesetzt. Diese Kombination kann im Rahmen der Erfindung ebenfalls verwendet werden. Bevorzugt ist es jedoch, das lineare polymere Biguanid und insbesondere PHMB in Kombination mit einem Tensid zu verwenden, wie es in der DE 10132817 A1 beschrieben ist. Unter den dort genannten Tensiden sind insbesondere die GlycinDerivate und von diesen die Amidoalkylbetaine einer Fettsäure bevorzugt. Besonders geeignet als Tenside zur Verwendung in der erfindungsgemäßen bioresorbierbaren Wundauflage sind Undecylenamidoalkylbetaine, Cocamidoalkylbetaine, Lauramidoalkylbetaine oder Ricinolamidoalkylbetaine, wobei der Alkylrest bevorzugt Ethyl oder Propyl ist. Derzeit besonders bevorzugt ist es, eine Kombination von PHMB bzw. dessen Hydrochlorid mit Undecylensäureamidopropylbetain als Tensid zu verwenden. Eine wässrige Lösung dieser Komponenten ist beispielsweise unter der Bezeichnung Prontosan® erhältlich. Für die Einarbeitung des Tensids in die bioresorbierbare Wundauflage gilt grundsätzlich das Gleiche, was für das polymere Biguanid ausgeführt wurde.

Die erfindungsgemäße bioresorbierbare Wundauflage auf Kollagen-Basis kann grundsätzlich in allen Anwendungsbereichen eingesetzt werden, in denen bereits bisher bioresorbierbare Wundauflagen eingesetzt wurden. Zu diesen Anwendungsbereichen zählen die bereits eingangs genannten Verwendungen als trockene oder feuchte Wundauflage. Abhängig von der Art der geplanten Verwendung können die Herkunft, der Typ und die Zubereitungsform des Kollagens in an sich bekannter Weise ausgewählt werden. Die Herstellung erfolgt grundsätzlich auf im Stand der Technik beschriebene Art und Weise aus tierischem Ausgangsmaterial, beispielweise aus Sehnen und/oder Häuten von Rindern, Schweinen oder Pferden, wobei equines Kollagen im Rahmen der Erfindung bevorzugt ist. Der Typ des verwendeten Kollagens ist ebenfalls nicht besonders beschränkt, jedoch sind die Typen I, III und X besonders geeignet.

Dichte und Vernetzungsgrad des Kollagens werden ebenfalls entsprechend den Anwendungsanforderungen gewählt. Wie bereits erwähnt, verlangsamt ein erhöhter Vernetzungsgrad die Resorption des Kollagens im Körper. Geeignete Dichten des Kollagens liegen zwischen 1 und 22 mg/cm³ und insbesondere zwischen 6 und 12 mg/cm³.

Die Zubereitungsform des Kollagens entspricht gleichfalls dem, was bereits aus dem Stand der Technik bekannt ist. Beispielsweise kann das Kollagen in Form eines porösen Schwamms, einer Folie, eines Films, einer Membran, eines Vlieses oder einer Tamponade vorliegen. Alle diese Zubereitungsformen können auf im Stand der Technik übliche Weise hergestellt werden. Vor der Verwendung wird das Kollagenmaterial vorzugsweise auf an sich bekannte Weise sterilisiert. Besonders geeignet hierfür sind die Sterilisation mit Ethylenoxid oder Gamma-Strahlung, vorzugsweise mit 28 kGray ± 10 %. Die Größe der Zubereitungsformen richtet sich nach der beabsichtigten Verwendung. Üblicherweise wird das Kollagenmaterial eine Dicke zwischen 0,1 und 20 mm und bevorzugt zwischen 2 und 7 mm aufweisen.

Bevorzugt weist die Wundauflage eine Aufnahmekapazität für Flüssigkeit von 0,1 ml/cm³ bis 1,0 ml/cm³ auf. Besonders bevorzugt liegt die Flüssigkeits-Aufnahmekapazität in einem Bereich von 0,2 ml/cm³ bis 0,5 ml/cm³.

Die erfindungsgemäße bioresorbierbare Wundauflage kann sowohl als trockene als auch als feuchte Wundauflage verwendet werden. In letzterem Fall, der bevorzugt ist, erfolgt die Herstellung zweckmäßig dadurch, dass das Kollagenmaterial mit einer wässrigen Lösung des linearen polymeren Biguanids und gegebenenfalls des Tensids getränkt wird. Hierzu werden die genannten Komponenten in einer für die beabsichtigte Wirkung geeigneten Menge in gereinigtem und sterilisierten Wasser gelöst. Dabei ist das als Biguanid besonders bevorzugte Polyhexamethylenbiguanid zweckmäßig in einer Konzentration von 0,01 bis 1 Gew.-%, insbesondere 0,01 bis 0,3 Gew.-% und bevorzugt 0,025 bis 0,1 Gew.-% in der wässrigen Lösung enthalten. Das Amphotensid und hier insbesondere Undecylensäureamidopropylbetain weist in der wässrigen Lösung zweckmäßig eine Konzentration von 0,01 bis 1,5 Gew.-%, insbesondere 0,01 bis 0,4 Gew.-% und bevorzugt 0,025 bis 0,1 Gew.-% auf.

Insgesamt wird die Konzentration des Polyhexamethylenbiguanids in der erfindungsgemäßen Wundauflage auf Kollagen-Basis zweckmäßig der beabsichtigten Indikation entsprechend bemessen. Die Konzentrationsbereiche sind dabei nicht besonders beschränkt, da bei einem Überschuss des Polyhexamethylenbiguanids praktisch keine nachteiligen Wirkungen beobachtet werden. Es ist daher in der Regel nur darauf zu achten, dass die Menge des Polyhexamethylenbiguanids ausreicht, die gewünschte prophylaktische und/oder therapeutische Wirkung zu erzielen. Die Konzentration des Tensids in der Wundauflage ist ebenfalls nicht besonders beschränkt. Die Menge wird so gewählt, dass die gewünschte Herabsetzung der Oberflächenspannung in dem zu behandelnden Körperbereich erzielt wird.

Gegenüber anderen mikrobiziden Wirkstoffen haben lineare polymere Biguanide wie insbesondere Polyhexamethylenbiguanid den Vorteil, dass sie über einen breiten pH-Bereich eingesetzt werden können, ohne das sie ihre bakterizide oder fungizide Wirksamkeit verlieren. Besonders gute Wirksamkeiten für die Wundheilung werden erzielt, wenn die erfindungsgemäße feuchte Wundauflage so eingestellt wird, dass der pH-Wert in einem Bereich von 3,5 bis 6,5 liegt.

Zusätzlich zu dem linearen polymeren Biguanid und gegebenenfalls einem Tensid kann die erfindungsgemäße bioresorbierbare Wundauflage weitere Komponenten wie insbesondere wenigstens einen pharmazeutischen Wirkstoff enthalten. Die Möglichkeit, Kollagen als Träger für pharmazeutische Wirkstoffe einzusetzen, ist grundsätzlich bereits beschrieben worden (vgl. die eingangs erwähnte DE 19503336 C2). Die erfindungsgemäß in Kollagenmaterial verwendeten linearen polymeren Biguanide haben den Vorteil, dass sie mit einer Vielzahl pharmazeutischer Wirkstoffe kompatibel sind und deren Wirksamkeit nicht beeinträchtigen Bevorzugt eingesetzt werden solche Wirkstoffe, die die Wundheilung fördern und/oder die Gefahr einer Wundinfektion reduzieren. Beispielhaft für derartige Wirkstoffe können Wachstumsfaktoren wie PDGF (Platelet derived growth factor) oder EGF (Epidermal growth factor), Cytokine, Hyaluronsäure oder Antibiotika genannt werden. Der Zusatz dieser Wirkstoffe zur erfindungsgemäßen bioresorbierbaren Wundauflage erfolgt in den üblichen pharmazeutisch wirksamen Dosen. Die Einarbeitung kann wie im Falle des Biguanids oder Tensids stattfinden.

Die Vorbereitung der Gewebe, mit denen die erfindungsgemäße bioresorbierbare Wundauflage in Kontakt kommt, kann auf die bisher übliche Weise erfolgen. Wunden werden zweckmäßig zuvor debridiert und gründlich ausgewaschen, wobei sich die Verwendung PHMB-haltiger Wundspüllösungen wie Prontosan® besonders anbietet. Die erfindungsgemäße Wundauflage kann aufgrund der erhöhten Resorptionsbeständigkeit 72 Stunden oder länger auf der Wunde verbleiben. Da das Infektionsrisiko minimal ist und die Wundauflage im Lauf der Zeit geliert und praktisch vollständig zu kurzkettigen Peptiden abgebaut wird, ist es nicht einmal erforderlich, die alte Wundauflage von der Wunde zu entfernen. Vielmehr kann eine weitere erfindungsgemäße Wundauflage auf die alte aufgebracht werden. Soll die alte Wundauflage entfernt werden, kann dies, falls erforderlich, unter Verwendung PHMB-haltiger oder anderer geeigneter Wundspüllösung geschehen. Anschließend wird die Wunde zweckmäßig erneut mit Wundspüllösung gereinigt, bevor die neue Wundauflage aufgebracht wird.

Die Erfindung wird nachfolgend anhand eines Herstellungsbeispiels für eine Wundauflage näher beschrieben.

### BEISPIEL 1

### Herstellung einer Wundauflage auf Kollagen-Basis

### a) Herstellung des Kollagenmaterials

Von allen Pigmentschichten und Muskelresten befreite frische Pferdesehnen werden homogenisiert. Eine Menge, die 100 g Trockengewicht entspricht, wird 24 Stunden lang in 3 I 0,05 M Citratpuffer (pH 3,7) extrahiert und anschließend gegen 1 %-ige Essigsäure 12 Stunden lang dialysiert. Das in 3 I 1%-iger Essigsäure suspendierte Gewebe wird 48 Stunden lang bei 15 °C unter ständigem Rühren mit Pepsin im Verhältnis Kollagen zu Pepsin = 50 : 1 inkubiert.
Der Ansatz wird mit 1 %-iger Essigsäure auf 5 I verdünnt und durch Zentrifugieren von den ungelösten Sehnenfragmenten befreit. Die viskose Kollagenlösung wird gegen alkalisiertes Leitungswasser (pH 8,0) diatysiert und dann scharf zentrifugiert. Der Rückstand wird erneut in 5 I 1%-iger Essigsäure gelöst und dialysiert. Dieser Vorgang wird mehrfach wiederholt. Anschließend wird mittels 0,05%-iger Essigsäure eine 1,5%-ige Kollagenlösung hergestellt.

### b) Herstellung der wirkstoffhaltigen Wundauflage

Das PHMB und gegebenenfalls das Tensid werden in die unter a) beschrieben Kollagenlösung in entsprechender Menge eingearbeitet. Diese visköse Lösung wird durch Lyophilisation oder eine andere, dem Stand der Wissenschaft entsprechende Methode durch Feuchtigkeitsentzug getrocknet. Als letztes wird der Schwamm verpackt und durch Ethylenoxid oder Gamma-Strahlung sterilisiert.

## Patentansprüche

1. Bioresorbierbare Wundauflage auf der Basis von Kollagen,
**dadurch gekennzeichnet,**
**dass** sie einen Gehalt an einem linearen polymeren Biguanid und/oder einem wasserlöslichen Salz desselben aufweist.

2. Bioresorbierbare Wundauflage gemäß Anspruch 1,
**dadurch gekennzeichnet,**
**dass** das lineare polymere Biguanid Polyhexamethylenbiguanid ist.

3. Bioresorbierbare Wundauflage gemäß Anspruch 1 oder 2,
**dadurch gekennzeichnet,**
**dass** sie weiter einen Gehalt an wenigstens einem Tensid aufweist.

4. Bioresorbierbare Wundauflage gemäß Anspruch 3,
**dadurch gekennzeichnet,**
**dass** das Tensid ein nicht-ionisches Tensid und/oder Amphotensid ist.

5. Bioresorbierbare Wundauflage gemäß Anspruch 4,
**dadurch gekennzeichnet,**
**dass** das Tensid ein Glycin-Derivat und insbesondere ein Amidoalkylbetain einer Fettsäure ist.

6. Bioresorbierbare Wundauflage gemäß Anspruch 5,
**dadurch gekennzeichnet,**
**dass** das Glycin-Derivat ein Undecylenamidoalkylbetain, Cocamidoalkylbetain, Lauramidoalkylbetain oder Ricinolamidoalkylbetain ist, wobei der Alkylrest bevorzugt Ethyl oder Propyl ist.

7. Bioresorbierbare Wundauflage gemäß einem der Ansprüche 1 bis 6,
**dadurch gekennzeichnet,**
**dass** das Kollagen ein equines Kollagen, bevorzugt vom Typ I, III oder X, ist.

8. Bioresorbierbare Wundauflage gemäß einem der Ansprüche 1 bis 7,
**dadurch gekennzeichnet,**
**dass** das Kollagen eine Dichte von 1 bis 22 mg/cm³ und insbesondere von 6 bis 12 mg/cm³ aufweist.

9. Bioresorbierbare Wundauflage gemäß einem der Ansprüche 1 bis 8,
**dadurch gekennzeichnet,**
**dass** das Kollagen die Form eines Schwamms, einer Folie, eines Films, einer Membran, eines Vlieses oder einer Tamponade aufweist.

10. Bioresorbierbare Wundauflage gemäß einem der Ansprüche 1 bis 9,
**dadurch gekennzeichnet,**
**dass** sie eine Flüssigkeits-Aufnahmekapazität von 0,1 ml/cm³ bis 1 ml/cm³ und insbesondere von 0,2 ml/cm³ bis 0,5 ml/cm³ aufweist.

11. Bioresorbierbare Wundauflage gemäß einem der Ansprüche 1 bis 10,
**dadurch gekennzeichnet,**
**dass** das Kollagen mit einer wässrigen Lösung des linearen polymeren Biguanids und gegebenenfalls des Tensids getränkt ist.

12. Bioresorbierbare Wundauflage gemäß Anspruch 11,
**dadurch gekennzeichnet,**
**dass** die Lösung
- 0,01 bis 1,0 Gew.-%, insbesondere 0,01 bis 0,3 Gew.-% und bevorzugt 0,025 bis 0,1 Gew.-% Polyhexamethylenbiguanid und
- 0,01 bis 1,5 Gew.%, insbesondere 0,03 bis 1 Gew.-%, bevorzugt 0,01 bis 0,4 Gew.-% und besonders bevorzugt 0,025 bis 0,1 Gew.-% eines Amphotensids und insbesondere von Undecylensäureamidopropylbetain enthält.

13. Bioresorbierbare Wundauflage gemäß einem der Ansprüche 1 bis 12,
**dadurch gekennzeichnet,**
**dass** sie als feuchte Wundauflage auf einen pH-Wert von 3,5 bis 6,5 eingestellt ist.

14. Bioresorbierbare Wundauflage gemäß einem der Ansprüche 1 bis 13,
**dadurch gekennzeichnet,**
**dass** sie einen Gehalt an wenigstens einem pharmazeutischen Wirkstoff aufweist, insbesondere wenigstens einen Wachstumsfaktor, ein Cytokin, Hyaluronsäure oder ein Antibiotikum.

## Claims

1. Bioresorbable collagen-based wound dressing, **characterized in that** it contains a linear polymer biguanide and/or a water-soluble salt thereof.

2. Bioresorbable wound dressing according to claim 1, **characterized in that** the linear polymer biguanide is polyhexamethylene biguanide.

3. Bioresorbable wound dressing according to claim 1 or 2, **characterized in that** it further contains at least one tenside.

4. Bioresorbable wound dressing according to claim 3, **characterized in that** the tenside is a non-ionic tenside and/or amphotenside.

5. Bioresorbable wound dressing according to claim 4, **characterized in that** the tenside is a glycine derivative and in particular an amidoalkyl betaine of a fatty acid.

6. Bioresorbable wound dressing according to claim 5, **characterized in that** the glycine derivative is undecylene amidoalkyl betaine, cocamidoalkyl betaine, lauramidoalkyl betaine or ricinolamidoalkyl betaine, wherein the alkyl residue is preferably ethyl or propyl.

7. Bioresorbable wound dressing according to one of claims 1 to 6, **characterized in that** the collagen is an equine collagen, preferably of type I, III, or X.

8. Bioresorbable wound dressing according to one of claims 1 to 7, **characterized in that** the collagen has a density from 1 to 22 mg/cm³ and in particular from 6 to 12 mg/cm³.

9. Bioresorbable wound dressing according to one of claims 1 to 8, **characterized in that** the collagen has the form of a sponge, a foil, a film, a membrane, a fibrous web or a tamponade.

10. Bioresorbable wound dressing according to one of claims 1 to 9, **characterized in that** the collagen has a liquid absorption capacity of 0.1 ml/cm³ to 1 ml/cm³ and in particular from 0.2 ml/cm³ to 0.5 ml/cm³.

11. Bioresorbable wound dressing according to one of claims 1 to 10, **characterized in that** the collagen is impregnated with an aqueous solution of the linear polymer biguanide and, optionally, the tenside.

12. Bioresorbable wound dressing according to claim 11, **characterized in that** the solution contains
- 0.01 to 1.0% by weight, in particular 0.01 to 0.3% by weight and preferably 0.025 to 0.1% by weight polyhexamethylene biguanide and
- 0.01 to 1.5% by weight, in particular 0.03 to 1% by weight, preferably 0.01 to 0.4% by weight and most preferably 0.025 to 0.1% by weight of an amphotenside and in particular of undecylenic acid amidopropyl betaine.

13. Bioresorbable wound dressing according to one of claims 1 to 12, **characterized in that** it is adjusted as a wet wound dressing to a pH value of from 3.5 to 6.5.

14. Bioresorbable wound dressing according to one of claims 1 to 13, **characterized in that** it contains at least one pharmaceutical agent, in particular at least a growth factor, a cytokine, hyaluronic acid or an antibiotic.

## Revendications

1. Pansement biorésorbable à base de collagène,
**caractérisé en qu'il**
présente une teneur en un polymère biguanide linéaire et/ou un sel soluble dans l'eau de ce même polymère.

2. Pansement biorésorbable selon la revendication 1,
**caractérisé en ce que**
ledit polymère biguanide linéaire est de la polyhexaméthylène biguanide.

3. Pansement biorésorbable selon la revendication 1 ou 2,
**caractérisé en ce qu'il**
présente de plus une teneur en au moins un agent tensioactif.

4. Pansement biorésorbable selon la revendication 3,
**caractérisé en ce que**
ledit agent tensioactif est un agent tensioactif non-ionique et/ou un agent de surface ampholytique.

5. Pansement biorésorbable selon la revendication 4,
**caractérisé en ce que**
ledit agent tensioactif est un dérivé de la glycine et en particulier une amidoalkylbétaine d'un acide gras.

6. Pansement biorésorbable selon la revendication 5,
**caractérisé en ce que**
ledit dérivé de la glycine est une undecylèneamido-alkylbétaine, cocamido-alkylbétaine, lauramido-alkylbétaine ou ricinolamido-alkylbétaine, le reste d'alkyle étant de préférence éthyle ou propyle.

7. Pansement biorésorbable selon une des revendications 1 à 6,
**caractérisé en ce que**
le collagène est un collagène équin, de préférence du type I, III ou X.

8. Pansement biorésorbable selon une des revendications 1 à 7,
**caractérisé en ce que**
le collagène présente une densité de 1 à 22 mg/cm³ et en particulier de 6 à 12 mg/cm³.

9. Pansement biorésorbable selon une des revendications 1 à 8,
**caractérisé en ce que**
le collagène présente la forme d'une éponge, d'une feuille, d'un film, d'une membrane, d'un matelas de fibres ou d'une tamponnade.

10. Pansement biorésorbable selon une des revendications 1 à 9,
**caractérisé en ce qu'il**
présente une capacité d'absorption de liquides de 0,1 ml/cm³ à 1 ml/cm³ et en particulier de 0,2 ml/cm³ à 0,5 ml/cm³.

11. Pansement biorésorbable selon une des revendications 1 à 10,
**caractérisé en ce que**
le collagène est imprégné d'une solution aqueuse du polymère biguanide et, le cas échéant, du agent tensioactif.

12. Pansement biorésorbable selon la revendication 11,
**caractérisé en ce que** ladite solution contient 0,01 à 1,0 % pond., en particulier
- 0,01 à 0,3 % pond. et de préférence 0,025 à 0,1 % pond, de polyhexaméthylène biguanide et
- 0,01 à 1,5 % pond., en particulier 0,03 à 1 % pond., de préférence 0,01 à 0,4 et en particulier de préférence 0,025 à 0,1 % pond, d'un agent tensioactif amphotère et en particulier de l'acide undecylène-amidopropyle-bétaine.

13. Pansement biorésorbable selon une des revendications 1 à 12,
**caractérisé en ce qu'il**
est ajusté comme pansement humide à un pH entre 3,5 et 6,5.

14. Pansement biorésorbable selon une des revendications 1 à 13,
**caractérisé en ce qu'il**
présente une teneur d'au moins une substance pharmaceutique, en particulier d'au moins un facteur de croissance, un cytokine, de l'acide hyaluronique ou un antibiotique.
